# EUROPEAN PATENT APPLICATION

(11) **EP 2 749 293 A1**
(43) Date of publication of application: **02.07.2014**
(21) Application number: 13199068.1
(22) Date of filing: 20.12.2013
(51) Int. Cl.: A61K 47/48, A61L 26/00, A61K 31/4172, A61K 31/715, A61K 31/765, C08L 1/00, C08L 3/00, C08L 5/00, C08L 29/02

(54) **Polymer composition and polymer material**

(30) Priority: 27.12.2012 TW 101150409
(71) Applicant: Industrial Technology Research Institute, Chutung Hsinchu 31040 (TW)
(72) Inventor: Shih, Ting-Yu, 116 Taipei City (TW); Teng, Tse-Min, 973 Hualien County (TW); Wang, Chia-Chun, 802 Kaohsiung City (TW); Chen, Yu-Hua, 300 Hsinchu City (TW); Chen, Jui-Hsiang, 300 Hsinchu City (TW); Yang, Mei-Ju, 300 Hsinchu City (TW); Chiang, Shu-Fang, 360 Miaoli County (TW); Chen, Yen-Chun, 300 Hsinchu City (TW); Chang, Chia-Ni, 350 Miaoli County (TW)
(74) Representative: Fuchs

(57) **Abstract**

A polymer composition including a polymer having a hydroxyl group and a histidine or a histidine derivative grafted to the polymer having a hydroxyl group. A polymer material is also provided, including a polymer composition which includes a polymer having a hydroxyl group, and a histidine or a histidine derivative grafted to the polymer having a hydroxyl group.

## Description

### TECHNICAL FIELD

The technical field relates to a polymer composition and a polymer material.

### BACKGROUND

It has been known that matrix metalloproteinases (MMPs) with high activity and concentration is a main reason for chronic wounds being hard to heal. Infection and inflammation of a wound will cause the tissue to secrete massive matrix metalloproteinases to decompose and remove dead bacterium and cells in the wound bed. However, it is found that overexpressed matrix metalloproteinase activities often cause decomposition of regenerate tissue and damage of growth factors, thus the wound become stuck in the inflammatory and proliferative stages, further resulting in a vicious circle that makes the wound hard to heal. Such chronic wounds are very common for wounds of diabetes patients and other pathogenic chronic disease patients, and can result in aggravation of the wound or even amputation. At the time, a dressing which has a matrix metalloproteinase inhibiting effect is capable of decreasing partial matrix metalloproteinase activity and promoting wound healing.

Moreover, in various inflammation responses, such as rheumatoid arthritis and osteoarthritis, there are also conditions in which tissues massively secrete matrix metalloproteinases. Matrix metalloproteinase at a high level of activity can often start various biochemical mechanisms; it is a pathogenic mechanism resulting in cartilage damage. Furthermore, activated matrix metalloproteinase resulting from coronary artery disease (such as coronary artery heart disease) and myocardium damage often further decomposes the extracellular matrix and causes plaque, and the vessel wall to become thinner or even break down. At this time materials having a matrix metalloproteinase inhibiting effect can be used to retard disease symptoms.

In addition, a synthesis of matrix metalloproteinase is usually needed during angiogenesis, cell migration and cell regeneration and reconstruction to help decomposition of the extracellular matrix. However, during tumor formation, over-expression of matrix metalloproteinase activity will cause decomposition of the extracellular basement membrane, and thus promote an increasing invasion ability and metastasis ability of tumors, and result in diffusion of tumor cells. At this time, development of inhibitors and materials having a matrix metalloproteinase inhibiting effect can help in decreasing angiogenesis and cell migration for tumors.

Matrix metalloproteinases is a group of polypeptide endonucleases which contain zinc ions and are capable of decomposing most of the extracellular matrix. The structure of matrix metalloproteinases consists of four domains, which are prodomain, catalytic center, hemopexin function domain, and transmembrane domain. Presently, more than 25 kinds of matrix metalloproteinases have been found, and they can be approximately divided into four types, comprising: (1) collagenases; (2) gelatinases; (3) stromelysins; and (4) membrane-type matrix metalloproteinases. Those can decompose gelatin, collagen and protein polysaccharide, etc. that abound in human tissue, and which relate to tissue formation, tissue metabolism and inflammatory response. During the secretion and generation period, the zinc atom of the activated position of a matrix metalloproteinase combines with and the cysteine, and the matrix metalloproteinase is inactivated, and after the polypeptide being digested and the zinc atom exposed, the matrix metalloproteinase is activated.

Presently, there are three main methods of inhibiting matrix metalloproteinases: (1) by using tissue inhibitor of metalloproteinase to combine with matrix metalloproteinase heme binding protein to form a reversible, noncovalent complex to make the matrix metalloproteinase lose activity; (2) by using a peptide or antibody to directly inhibit matrix metalloproteinase activity expression; (3) by using a molecule that has a high affinity among zinc ions to bond to the matrix metalloproteinase to make the matrix metalloproteinase inactive, wherein the mechanism for the molecule chelating zinc ions to inactivate matrix metalloproteinase is through atoms with lone pair electrons, such as phosphorous, nitrogen, sulfur, and oxygen, which is very easy to form a coordination form with a transition metal.

Literature and the development of related products shows that advanced dressings for chronic wounds require to inhibit matrix metalloproteinase activity to promote reconstruction and healing of the wound bed. However, inhibitory effects of currently available medical devices are only temporary due to that the heavy loaded of wound fluid easily carry inhibitors away. Therefore techniques of immobilized molecules with inhibitory function might be able to prolong the effective period of time.

At present a new material which is capable of inhibiting matrix metalloproteinase activity for the long term is needed.

### SUMMARY

The disclosure provides a polymer composition, comprising: a polymer having a hydroxyl group; and a histidine or a histidine derivative grafted to the polymer having a hydroxyl group.

The disclosure also provides a polymer material, comprising: a polymer composition, which comprises: a polymer having a hydroxyl group; and a histidine or a histidine derivative grafted to the polymer having a hydroxyl group.

The disclosure further provides a medical device, comprising: a polymer composition, which comprises: a polymer having a hydroxyl group; and a histidine or a histidine derivative grafted to the polymer having a hydroxyl group; and a substrate.

In particular, the polymer material according to the invention comprises a polymer composition as described in any one of claims 1-17. The polymer material can be such that the polymer composition forms a solution. Said solution can be directly processed to a bulk material.

The medical device according to the present invention comprises, for example, a polymer composition as claimed in any one of claims 1-17; and a substrate, wherein the polymer composition and the substrate are intermixed to form the medical device, or the polymer composition physically adheres to a surface of the substrate or to the inside of the substrate to form the medical device. Furthermore, the medical device can be such that a material of the substrate comprises polysaccharides, polyurethanes, polyvinyl alcohols (PVA), poly(ethylene vinyl-co-alcohol) (EVOH), polypropylenes or a combination thereof. The medical device can comprise a wound dressing, a tissue substitute, a tissue engineering scaffold, a blood-contacting device or a catheter.

A detailed description is given in the following embodiments with reference to the accompanying drawings.

### BRIEF DESCRIPTION OF DRAWINGS

The embodiments of disclosure can be more fully understood by reading the subsequent detailed description and examples with references made to the accompanying drawings, wherein:
FIG. 1 shows one embodiment for forming the polymer composition of the present disclosure;
FIG. 2 shows an application manner for the polymer composition of the present disclosure;
FIG. 3 shows inhibitory rates of Boc-histidine with different concentrations on matrix metalloproteinase-9;
FIG. 4 shows inhibitory rates of histidine grafted-polyvinyl alcohol derivatives, "PVA-g-BocHis", (Lot 1 of Example 1) with different concentrations on matrix metalloproteinase-9;
FIG. 5 shows an inhibitory rate of a film formed from histidine grafted-polyvinyl alcohol derivative, "PVA-g-BocHis", (Lot 1 of Example 1) on matrix metalloproteinase-9;
FIG. 6 shows an inhibitory rate of a film formed from histidine grafted-polyvinyl alcohol derivative, "PVA-g-BocHis", (Lot 2 of Example 1) on matrix metalloproteinase-9;
FIG. 7 shows inhibitory rates of films Lot 1-1 and Lot 1-2 formed from histidine grafted-polyvinyl alcohol derivative, "PVA-g-BocHis", (Lot 2 of Example 1) on matrix metalloproteinase-9;
FIG. 8 shows inhibitory rates of films formed from histidine grafted-poly(ethylene vinyl-co-alcohol) derivative, "EVOH-g-BocHis", (Lot 3 and Lot 4 of Example 2) on matrix metalloproteinase-9;
FIG. 9 shows an inhibitory rate of a film formed from histidine grafted-hydroxypropyl methylcellulose derivative, "HPMC-g-BocHis", (Lot 5 of Example 3) on matrix metalloproteinase-9;
FIG. 10 shows an inhibitory rate of a film formed from histidine grafted-hydroxypropyl methylcellulose derivative, "HPMC-g-BocHis", (Lot 6 of Example 3) on matrix metalloproteinase-9;
FIG. 11 shows an inhibitory rate of a film formed from histidine grafted-hydroxypropyl methylcellulose derivative, "HPMC-g-BocHis", (Lot 6 and Lot 7 of Example 3) on matrix metalloproteinase-9;
FIG. 12 shows the results of an evaluation of the long-term inhibiting effect of histidine grafted-poly(ethylene vinyl-co-alcohol) derivative film sample (Lot 3) on matrix metalloproteinase-9;
FIG. 13 shows the results of an evaluation of the long-term inhibiting effect of histidine socking treated substrate (EVOH-c-BocHis) and histidine grafted-poly(ethylene vinyl-co-alcohol) derivative film sample (Lot 4); and
FIG. 14 shows the results of an evaluation of the effect of histidine grafted derivative film samples on the activity of matrix metalloproteinase-9 in the wound effusion fluid of a diabetic rat.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

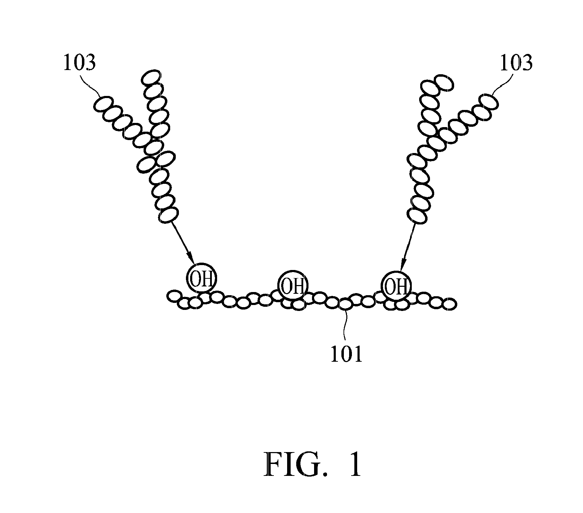

One embodiment of the disclosure provides a polymer composition, which has zinc ion affinity, and has an effect of inhibiting matrix metalloproteinases (MMPs) activity. See FIG. 1. FIG. 1 shows one embodiment for forming the polymer composition of the present disclosure. In FIG. 1, it is shown that a histidine or a histidine derivative 103 is grafted to a polymer having a hydroxyl group 101 to form the polymer composition of the present disclosure.

According to FIG. 1, it is known that the polymer composition of the present disclosure may comprises a polymer having a hydroxyl group 101 and a histidine or a histidine derivative 103 grafted to the polymer having a hydroxyl group. The polymer composition of the present disclosure has zinc ion affinity, and has an effect of inhibiting matrix metalloproteinases (MMPs) activity. In one embodiment, matrix metalloproteinase, which can be inhibited by the polymer composition of the present disclosure, may comprise but is not limited to matrix metalloproteinase-1 (MMP-1), matrix metalloproteinase-2 (MMP-2), matrix metalloproteinase-8 (MMP-8), matrix metalloproteinase-9 (MMP-9) and/or matrix metalloproteinase-13 (MMP-13).

In the polymer composition of the present disclosure, the histidine or the histidine derivative accounts for about 0.1 to 99 wt% of the polymer composition.

In the polymer composition of the present disclosure, the polymer having a hydroxyl group may comprise a synthetic polymer or a natural polymer, and the synthetic polymer or the natural polymer mentioned above may comprise a linear polymer or a branched polymer.

In one embodiment, the linear polymer or the branched polymer mentioned above may be a linear synthetic polymer. Examples of the foregoing linear synthetic polymer may comprise polyalkylene glycol, polyvinyl alcohol (PVA), polyvinyl acetate (PVAc), poly(vinyl alcohol-co-vinyl acetate), poly(ethylene vinyl-co-alcohol), (EVOH), a derivative thereof and a combination thereof, etc., but it is not limited thereto.

Moreover, in one embodiment, in the polymer composition of the present disclosure, the polymer having a hydroxyl group mentioned above may be a natural polymer. The natural polymer may comprise a polysaccharide polymer. A polysaccharide polymer which is suitable for use in the polymer composition may comprise hyaluronic acid, starch, cellulose, methylcellulose, hydroxypropyl cellulose, hydroxypropyl methylcellulose, oxidized cellulose, dextran, scleroglucan, chitin, chitosan, curdlan, alginate, carrageenan, pectin, gum Arabic, guar gum, gellan, pullulan, chondroitin sulfate, heparin, keratin sulfate or a derivative thereof, etc.

In the polymer composition of the present disclosure, since the histidine or the histidine derivative has a nitrogen retaining a lone pair, the histidine or the histidine derivative is capable of having the effect of chelating a zinc ion. The histidine derivative may comprise a *N*^{α}-protected histidine derivative, but is not limited thereto. Examples of the foregoing *N*^{α}-protected histidine derivative may comprise, but are not limited to, *N*^{α}-Boc-histidine, *N*^{α}-Cbz-histidine, *N*^{α}-Fmoc-histidine and *N*^{α}-Ac-histidine, etc.

In the polymer composition of the present disclosure, the histidine or the histidine derivative may be directly grafted to the polymer having a hydroxyl group, or may be grafted to the polymer having a hydroxyl group by a spacer. The spacer may comprise, but is not limited to alkyl carbon chains, phenyl group, polyethyleneoxide (PEO), polyethylglycol (PEG), or polypropylene oxide (PPO) repeating units.

In one embodiment, the histidine or the histidine derivative is directly grafted to the polymer having a hydroxyl group. In this embodiment, the foregoing histidine or the foregoing histidine derivative may be directly grafted to the polymer having a hydroxyl group through a chemical covalent bond, and the chemical covalent bond mentioned above may comprise an ester bond or a urethane, but is not limited thereto. The ester bond replaces the histidine or a functional group capable of protonizing the histidine at the hydroxyl group of the polymer having a hydroxyl group.

In a specific embodiment, the polymer composition of the present disclosure comprises a polymer having a hydroxyl group and a histidine or a histidine derivative grafted to the polymer having a hydroxyl group, wherein the foregoing polymer having a hydroxyl group comprises polyvinyl alcohol (PVA), poly(ethylene vinyl-co-alcohol) (EVOH), hyaluronic acid, cellulose or hydroxypropyl methylcellulose, and the foregoing histidine derivative is *N*^{α}-Boc-histidine.

In another embodiment, the foregoing histidine or the foregoing histidine derivative may be grafted to the polymer having a hydroxyl group by a spacer, and examples for a spacer which are suitable for the disclosure may comprise alkyl carbon chains, phenyl group, polyethyleneoxide (PEO), polyethylglycol (PEG), or polypropylene oxide (PPO) repeating units, etc., but they are not limited thereto.

The inhibitory rate of the polymer composition of the present disclosure on a matrix metalloproteinase can reach 10 to 100%. In one embodiment, the inhibitory rate of the polymer composition of the present disclosure on matrix metalloproteinase-9 can reach about 20.39% to 62.15%.

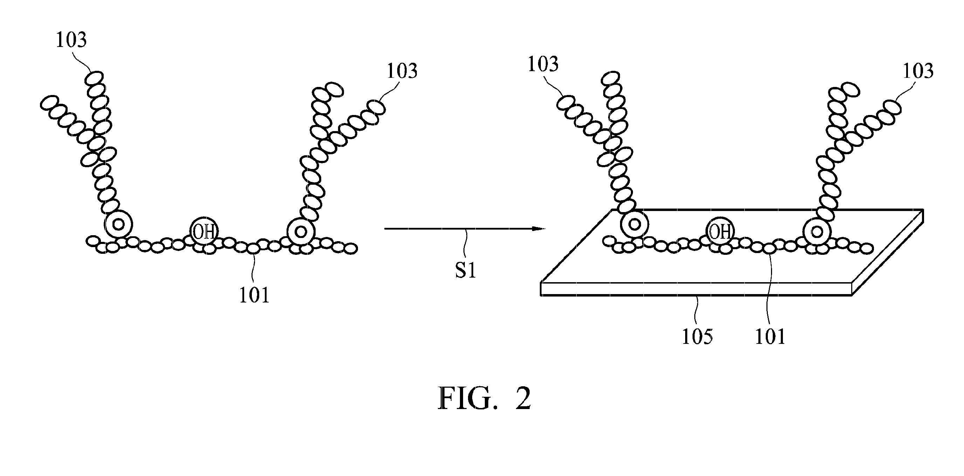

For the manner of application of the polymer composition of the present disclosure, refer to FIG. 2, but this is not meant to be limiting. FIG. 2 shows that the polymer composition of the present disclosure containing a polymer having a hydroxyl group 101 and a histidine or a histidine derivative 103 grafted to the polymer having a hydroxyl group 101 is combined with a substrate 105 to be used through an intermixing, coating or soaking process S 1.

The polymer composition of the present disclosure may be dispersed in a substrate, or may be dispersed on a surface and/or the inside of a medical device, in an intermixing manner.

Alternatively, the polymer composition of the present disclosure may form a solution. In one embodiment, the solution mentioned above may be directly processed to a bulk material, and the bulk material may be used in a medical application, but it is not limited thereto.

In another embodiment, the solution mentioned above may be used to treat a substrate or a medical device to physically adhere on a surface of the substrate or a surface of the medical device. In this embodiment, a material of the substrate may comprise, but is not limited to, polysaccharides (such as cellulose and a derivative thereof, cellulose and a derivative thereof, hyaluronic acid and a derivative thereof, etc.), polyurethanes, polyvinyl alcohols (PVA), poly(ethylene vinyl-co-alcohol) (EVOH), polypropylenes, etc. or a combination thereof, and examples of the medical device may comprise a wound dressing, a tissue substitute, a tissue engineering scaffold, a blood-contacting device and a catheter, etc., but it is not limited thereto.

Another embodiment of the present disclosure further provides a polymer material which comprises the polymer composition of the present disclosure mentioned above, and the polymer composition of the present disclosure mentioned above has zinc ion affinity, and has an effect of inhibiting matrix metalloproteinases (MMPs) activity. Since the polymer material of the present disclosure contains the polymer composition having an effect for inhibiting matrix metalloproteinases (MMPs) activity, the polymer material of the present disclosure may be used for a medical use to improve symptoms of tissue inflammation of a patient and/or to promote wound healing, but is not limited thereto.

Since the polymer material of the present disclosure contains the polymer composition having an effect for inhibiting matrix metalloproteinases (MMPs) activity, the polymer material of the present disclosure may be applied to medical devices such as catheter, stents, guidewire, or scaffold to ease symptoms of a coronary heart disease or stroke patient, but is not limited thereto.

In one embodiment, the polymer composition of the polymer material may form a solution. Furthermore, the solution may be directly processed to a bulk material.

Further another embodiment of the present disclosure provides a medical device. The medical device may comprise the polymer composition of the present disclosure mentioned above and a substrate.

In one embodiment, the polymer composition and the substrate may be intermixed to form the medical device, or the polymer composition physically adheres to a surface of the substrate or to the inside of the substrate to form the medical device.

Examples of the material of the substrate mentioned above may be polysaccharides, polyurethanes, polyvinyl alcohols (PVA), poly(ethylene vinyl-co-alcohol) (EVOH), polypropylenes, etc. or a combination thereof. In addition, the aforementioned medical device may comprise, but is not limited to, a wound dressing, a tissue substitute, a tissue engineering scaffold, a blood-contacting device or a catheter, etc.

### EXAMPLES

### Example 1

### Synthesis of a histidine grafted-polyvinyl alcohol derivative, "PVA-g-BocHis"

The structure of the histidine grafted-polyvinyl alcohol derivative, "PVA-g-BocHis" is shown as Formula (I) in the following:

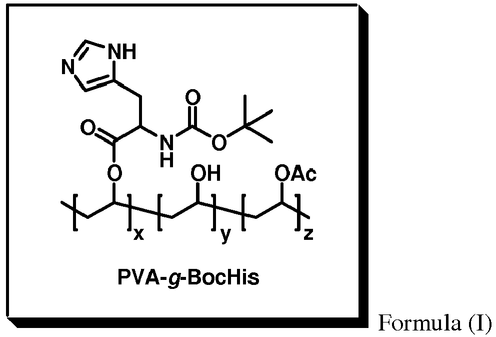

Boc-His-OH (4.48 g, 17.57 mmol) and DMAP (1.95 g, 15.97 mmol) were placed in a two-neck flask containing a magnetic stirrer therein. The two-neck flask was vacuumized for 3 minutes to remove the air therein and then the two-neck flask was filled with dry nitrogen. Next, DMAc (35 ml) was added into the two-neck flask and stirred for 10 minutes until the contents of the two-neck flask were uniformly suspended. EDC solid (3.06 g, 15.97 mmol) was quickly poured into the two-neck flask, and the two-neck flask was placed in a 30°C water bath for 3 hours for a reaction to activate Boc-His-OH. PVA₁₀ₖ (Molecular weight is 10000) (2.79 g, 53.24 mmol, 80% hydrolyzed) was added to DMAc (28 ml) and stirred at 80°C to completely dissolve to form a PVA solution, and after that the PVA solution was cooled down 45°C to ready for use. The activated Boc-His-LG solution (LG = Leaving Group) was quickly added to the PVA solution mentioned above, and the reaction was continued at 45°C for 24 hours to form a reactive solution. After being cooled down naturally, the reactive solution mentioned above was packaged in a dialysis bag (Molecular weight cut off value, MWCO: 6-8,000), dialyzed with DMAc (1.5 L, 20X) for 40 hours (the dialysis solution was exchanged one time at 16 hours), and then dialyzed with DIW (7.5 L, 100X) for 48 hours (the dialysis solution was exchanged at 3, 6, 9, 12, 24, 27, 30, 33 and 36 hours). The resulting solid was collected and lyophilized to obtain a product, PVA-g-BocHis.

According to the preceding experimental method, by adjusting reactive equivalents between PVA and Boc-His-OH, different lots of different polymer materials, PVA-g-BocHis, with different degrees of grafting BocHis were obtained, and the different lots of different polymer material had their grafting ratios determined by nuclear magnetic resonance spectroscopy. The results are shown in Table 1.

**Table 1: Material specifications for PVA-g-BocHis**

| Lots | Molecular weight of PVA | Feed ratio of Boc-His-OH/PVA | BocHis grafting ratio | BocHis wt% |
|---|---|---|---|---|
| 1 | 10,000 | 0.3/1 | 13.3% | 39 wt% |
| 2 | 10,000 | 1/1 | 19.0% | 49 wt% |

### Example 2

### Synthesis of a histidine grafted-poly(ethylene vinyl-co-alcohol), "EVOH-g-BocHis"

The structure of the histidine grafted-poly(ethylene vinyl-co-alcohol) derivative, "EVOH-g-BocHis" is shown as Formula (II) in the following:

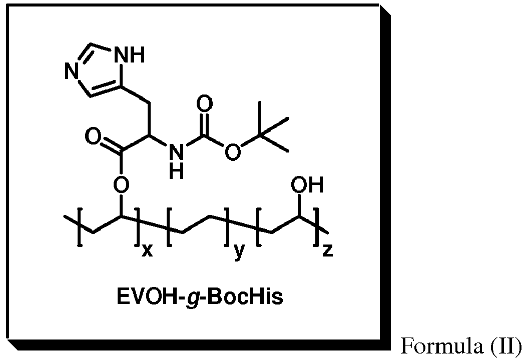

Boc-His-OH (11.46 g, 44.88 mmol) and DMAP (4.98 g, 40.8 mmol) were placed in a two-neck flask containing a magnetic stirrer therein. The two-neck flask was vacuumized for 3 minutes to remove the air therein and then the two-neck flask was filled with dry nitrogen. Next, DMAc (90 ml) was added into the two-neck flask and stirred for 10 minutes until the contents of the two-neck flask were uniformly suspended. EDC solid (7.82 g, 40.8 mmol) was quickly poured into the two-neck flask, and the two-neck flask was placed in a 30°C water bath for 3 hours for a reaction to activate Boc-His-OH. EVOH (5.84 g, 150 mmol, 32 mol% ethylene unit) was added to DMAc (58 ml) and stirred at 80°C to completely dissolve to form a EVOH solution, and after that the EVOH solution was cooled down 45°C to ready for use. The activated Boc-His-LG solution (LG = Leaving Group) was quickly added to the EVOH solution mentioned above, and the reaction was continued at 45°C for 24 hours to form a reactive solution. After being cooled down naturally, the reactive solution mentioned above was packaged in a dialysis bag (MWCO: 6-8,000), dialyzed with DMAc (3.0 L, 20X) for 40 hours (the dialysis solution was exchanged one time at 16 hours). After that DIW (5.0 L, 25X) was poured into the liquid in the dialysis bag to perform a first reprecipitation. The resulting solid was re dissolved with MeOH (10 % w/v) to form a solution, and then DIW (7.0 L, 35X) was poured into the solution to perform a second reprecipitation. The resulting solid was collected, spread to be washed with DIW three times, and then lyophilized to obtain a product, EVOH-g-BocHis.

According to the preceding experimental method, by adjusting reactive equivalents between EVOH and Boc-His-OH, different lots of different polymer materials, EVOH-g-BocHis, with different degrees of grafting BocHis were obtained, and the different lots of different polymer material had their grafting ratios determined by nuclear magnetic resonance spectroscopy. The results are shown in Table 2.

**Table 2: Material specifications for EVOH-g-BocHis**

| Lots | EVOH | Feed ratio of Boc-His-OH/EVOH | BocHis grafting ratio | BocHis wt% |
|---|---|---|---|---|
| 3 | EV3251 | 0.4/1 | 13% | 45 wt% |
| 4 | EV3251 | 0.4/1 | 15% | 49 wt% |

### Example 3

### Synthesis of a histidine grafted-hydroxypropyl methylcellulose, "HPMC-g-BocHis"

The structure of the histidine grafted- hydroxypropyl methylcellulose derivative, "HPMC-g-BocHis" is shown as Formula (III) in the following:

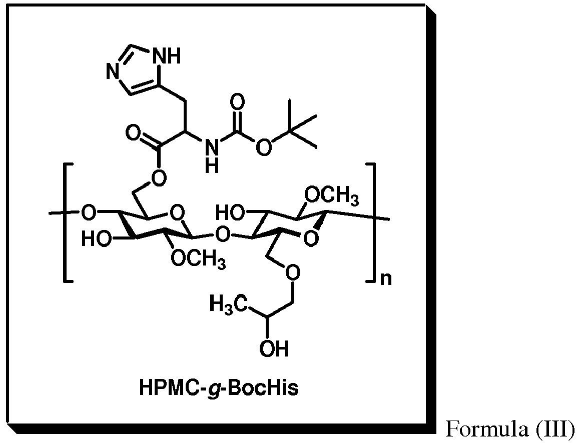

Boc-His-OH (2.92 g, 11.44 mmol) and DMAP (1.27 g, 10.4 mmol) were placed in a two-neck flask containing a magnetic stirrer therein. The two-neck flask was vacuumized for 3 minutes to remove the air therein and then the two-neck flask was filled with dry nitrogen. Next, DMAc (22.9 ml) was added into the two-neck flask and stirred for 10 minutes until the contents of the two-neck flask were uniformly suspended. EDC solid (1.99 g, 10.4 mmol) was quickly poured into the two-neck flask, and the two-neck flask was placed in a 30°C water bath for 3 hours for a reaction to activate Boc-His-OH. HPMC (2.0 g, 10.4 mmol, Mn 120,000, DS of methoxyl.1-1.6 mol, MS of propylene oxide 0.1-0.3 mol) was added to DMAc (40 ml) and stirred at 80°C to completely dissolve to form a HPMC solution, and after that the HPMC solution was cooled down 50°C to ready for use. The activated Boc-His-LG solution (LG = Leaving Group) was quickly added to the PVA solution mentioned above, and the reaction was continued at 50°C for 24 hours to form a reactive solution. After being cooled down naturally, the reactive solution mentioned above was packaged in a dialysis bag (MWCO: 6-8,000), dialyzed with DMAc (1.4 L, 20X) for 40 hours (the dialysis solution was exchanged one time at 16 hours), and then dialyzed with DIW (7.0 L, 100X) for 72 hours (the dialysis solution was exchanged at 3, 6, 9, 12, 24, 27, 30, 33, 36, 48, 52 and 56 hours). The resulting solid was collected and lyophilized to obtain a product, HPMC-g-BocHis.

According to the preceding experimental method, by adjusting reactive equivalents between HPMC and Boc-His-OH, different lots of different polymer materials, HPMC-g-BocHis, with different degrees of grafting BocHis were obtained, and the different lots of different polymer material had their grafting ratios determined by nuclear magnetic resonance spectroscopy. The results are shown in Table 3.

**Table 3: Material specifications for HPMC-g-BocHis**

| Lots | Molecular weight of HPMC | Feed ratio of Boc-His-OH/HPMC | BocHis grafting ratio | BocHis wt% |
|---|---|---|---|---|
| 5 | 120,000 | 1/1 | 11.4% | 13 wt% |
| 6 | 120,000 | 1/1 | 18.0% | 20 wt% |
| 7 | 120,000 | 1/1 | 16.0% | 18 wt% |

### Example 4

### Synthesis of a histidine grafted-hyaluronic acid, "HA-g-BocHis"

The structure of the histidine grafted-hyaluronic acid derivative, "HA-g-BocHis" is shown as Formula (IV) in the following:

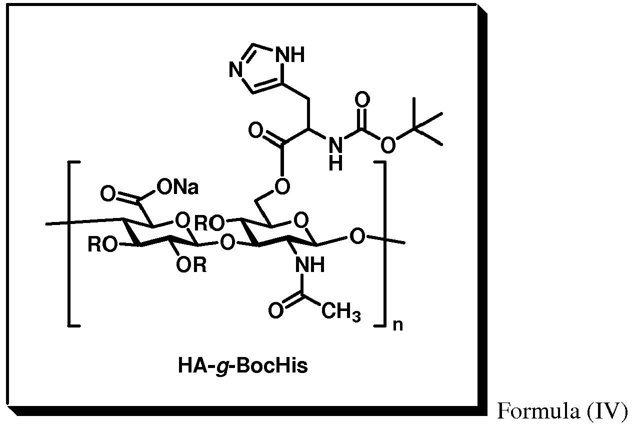

Boc-His-OH (8.42 g, 33.0 mmol) and DMAP (3.67 g, 30.0 mmol) were placed in a two-neck flask containing a magnetic stirrer therein. The two-neck flask was vacuumized for 3 minutes to remove the air therein and then the two-neck flask was filled with dry nitrogen. Next, DMAc (66 ml) was added into the two-neck flask and stirred for 10 minutes until the contents of the two-neck flask were uniformly suspended. EDC solid (5.75 g, 30.0 mmol) was quickly poured into the two-neck flask, and the two-neck flask was placed in a 30°C water bath for 3 hours for a reaction to activate Boc-His-OH. HATBA (18.6 g, 30.0 mmol) was poured into a glass reaction tank. The glass reaction tank was vacuumized for 10 minutes to remove the air therein and then the glass reaction tank was filled with dry nitrogen. DMAc which had been dewatered by a molecular sieve (186 ml) was added into the glass reaction tank and placed in a 45°C recurring water bath and stirred by the mechanical stirring device at 250 rpm for more than 2 hours to ensure HA₁₆₀₀₀TBA (molecular weight is 1600) or HA₃₅₀₀₀₀TBA (molecular weight is 350,000) was uniformly dissolved to form an HA solution. The activated Boc-His-LG solution (LG = Leaving Group) was quickly added to the glass reaction tank containing the HATBA solution. After 30 minutes, the rotation rate of the mechanical stirring device in the glass reaction tank was increased to 300 rpm, and the reaction of the solution in the glass reaction tank was continued at 45°C for 24 hours to form a reactive solution. After being cooled down naturally, the reactive solution mentioned above was packaged in a dialysis bag (MWCO: 12-14,000), dialyzed with DMAc (6.0 L, 20X) for 40 hours (the dialysis solution was exchanged one time at 16 hours), and then dialyzed with DIW (18.0 L, 100X) for 72 hours (the dialysis solution was exchanged at 3, 6, 9, 12, 24, 27, 30, 33, 36, 48, 52 and 56 hours). Next, the aqueous solution as collected and passed through sodium ion exchange resin (ROHM HAAS, food level, 520 g), and TBA was replaced with sodium ion, and the aqueous solution was concentrated in vacuum to a concentration of about 1-1.5 wt%. After that, the aqueous solution was adjusted pH value to 7.6±0.2 with 0.1 M NaOH, and lyophilized to obtain a product, HA-g-BocHis.

According to the preceding experimental method, by adjusting reactive equivalents between HA and Boc-His-OH, different lots of different polymer materials, HA-g-BocHis, with different degrees of grafting BocHis were obtained, and the different lots of different polymer material had their grafting ratios determined by nuclear magnetic resonance spectroscopy. The results are shown in Table 4.

**Table 4: Material specifications for HA-g-BocHis**

| Lots | Molecular weight of HA | Feed ratio of Boc-His-OH/HA | BocHis grafting ratio | BocHis wt% |
|---|---|---|---|---|
| 8 | 16,000 | 0.4/1 | 17% | 10 wt% |
| 9 | 16,000 | 1/1 | 44% | 22 wt% |
| 10 | 16,000 | 2/1 | 71% | 31 wt% |
| 11 | 350,000 | 1/1 | 19% | 11 wt% |

### Example 5

### Synthesis of a histidine grafted-hyaluronic acid, "Cellulose-g-BocHis"

The structure of the histidine grafted-cellulose derivative, "Cellulose-g-BocHis" is shown as Formula (V) in the following:

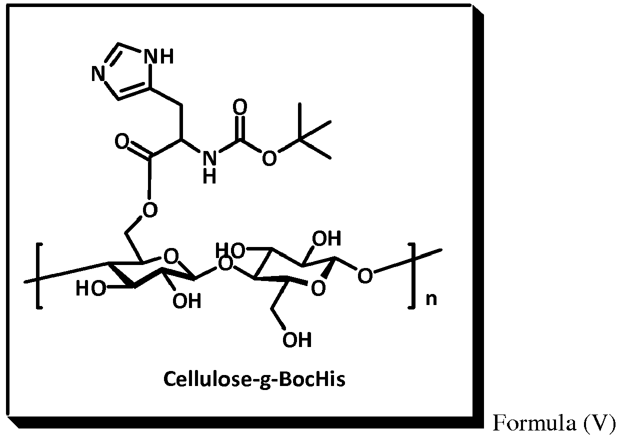

Boc-His-OH (1.39 g, 5.34 mmol) and DMAP (0.6 g, 4.94 mmol) were placed in a two-neck flask containing a magnetic stirrer therein. The two-neck flask was vacuumized for 3 minutes to remove the air therein and then the two-neck flask was filled with dry nitrogen. Next, DMAc (10.9 ml) was added into the two-neck flask and stirred for 10 minutes until the contents of the two-neck flask were uniformly suspended. EDC solid (0.95 g, 4.94 mmol) was quickly poured into the two-neck flask, and the two-neck flask was placed in a 30°C water bath for 3 hours for a reaction to activate Boc-His-OH. Cellulose fabric (2.00g, 12.34 mmol) was placed into a 150 ml glass reaction tank, soaked in DMAc which had been dewatered by a molecular sieve (20 ml) and stirred at 45°C at 100 rpm for more than 2 hours. The activated Boc-His-LG solution (LG = Leaving Group) was quickly added to the glass reaction tank to make cellulose fabric continuously react with Boc-His-LG solution at 45°C for 24 hours. After being cooled down naturally, the reacted cellulose fabric was soak in DMAc (0.5 L, 20X) and stirred for 0.5 hours, and then placed in DIW (1.0 L, 100X) and stirred for 1 hour. After that, the reacted cellulose fabric was washed with DIW three times to remove impurity produced after the reaction. Finally, the reacted cellulose fabric was dried to obtain Cellulose-g-BocHis.

### Example 6

### Inhibition test of matrix metalloproteinase-9 activity for different samples

### Analysis method

### Step 1: Activation of pro-matrix metalloproteinase-9

100 nM APMA (final concentration 1 M) as added to a 10 µg/ml of pro-matrix metalloproteinase-9 solution (R&D), and reacted in a 37°C incubator for 2 hours to convert pro matrix metalloproteinase-9 to activated pro-matrix metalloproteinase-9

### Step 2: Test samples

The activated pro-matrix metalloproteinase-9 was diluted with matrix metalloproteinase-9 activity analysis buffer (50mM Tris, 10 mM CaCl₂, 150 mM NaCl, 0.05% Brij-35(w/v), pH 7.5) to a concentration of 10 ng/ml. Activated pro-matrix metalloproteinase-9 was added to each test sample and placed in a 37°C incubator for reaction for 2 or 24 hours. The negative control group contained no activated pro-matrix metalloproteinase-9 solution to test the response of the sample, and positive control added metalloproteinase-9 activity inhibitor, 1,10-Phenanthroline (1,10-PT) (final concentration: 0.1 mM), to the activated pro-matrix metalloproteinase-9 solution.

### Step 3: Analysis for matrix metalloproteinase-9

The activated pro-matrix metalloproteinase-9 solution after being reacted with the material was taken in a 96-well black plate. Equal volume of substrate for matrix metalloproteinase-9 was added to the 96-well black plate and reacted in a 37°C incubator for 0.5-1 hour, and then the fluorescence (Ex/Em=320/405 nm) of the reaction was determined by a high sensitivity fluorescence reader (Flexstation3).

### Experimental results

### A. Effect of Boc-histidine concentration on inhibition of matrix metalloproteinase-9 activity

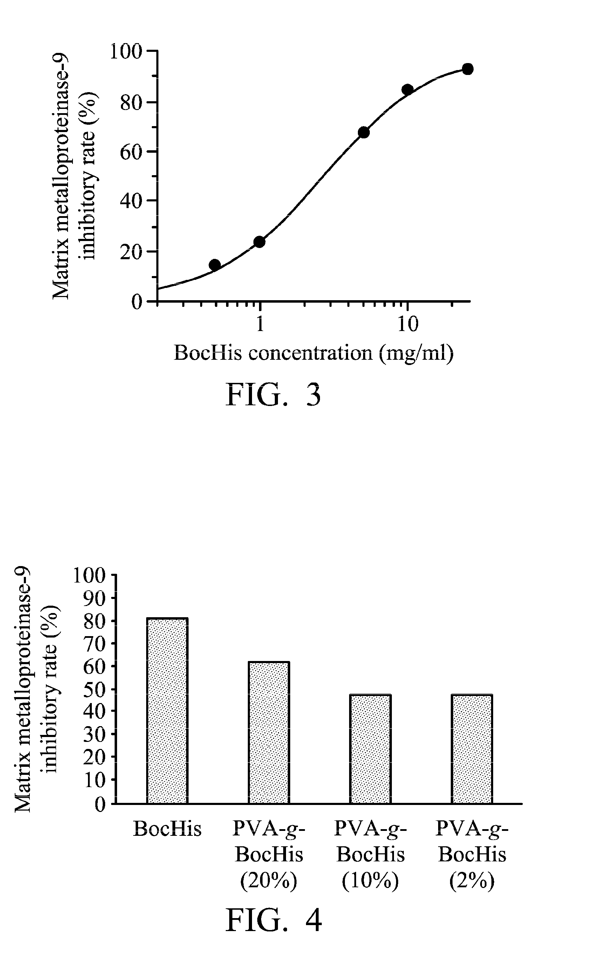

Boc-histidine with different concentrations which were known were reacted with the activated pro-matrix metalloproteinase-9, respectively for 2 hours, and variation of the effect of Boc-histidine with different concentrations on activated matrix metalloproteinase-9 activity was determined. The results were shown in FIG. 3. When the concentration of Boc-histidine reached 2.64 mg/ml, Boc-histidine had 50% inhibitory effect on matrix metalloproteinase-9. y = 21.83ln(x) + 28.06.

### B. Evaluation of inhibitory effect of powder of Boc-histidine grafted-polyvinyl alcohol derivative, "PVA-g-BocHis", on matrix metalloproteinase-9 activity

Powder of Boc-histidine grafted-polyvinyl alcohol derivative, "PVA-g-BocHis", with 13.3% of grafting ratio (Lot 1 of Example 1) was prepared as test samples with different concentrations, and the effects thereof on matrix metalloproteinase-9 activity were tested. The results are shown as Table 5 and FIG. 4.

**Table 5: Inhibitory effect of Boc-histidine grafted-polyvinyl alcohol derivative on matrix metalloproteinase-9**

| Sample | BocHis | PVA-*g*-BocHis (20%) | PVA-*g*-BocHis (10%) | PVA-*g*-BocHis (2%) |
|---|---|---|---|---|
| Matrix metalloproteinase-9 inhibitory rate (%) | 81.05 | 62.15 | 47.78 | 47.89 |

The results showed that when the grafting ratio for PVA-g-BocHis was 13.3%, and the concentration of PVA-g-BocHis was 2%∼10%, PVA-g-BocHis had an inhibitory rate of 47.78% to matrix metalloproteinase-9; when the concentration of PVA-g-BocHis was 20%, PVA-g-BocHis had an inhibitory rate of 62.15% to matrix metalloproteinase-9.

C. Evaluation of inhibitory effect of a film of histidine grafted-polyvinyl alcohol derivative, "PVA-g-BocHis", of Lot 1 of Example 1 on matrix metalloproteinase-9 activity.

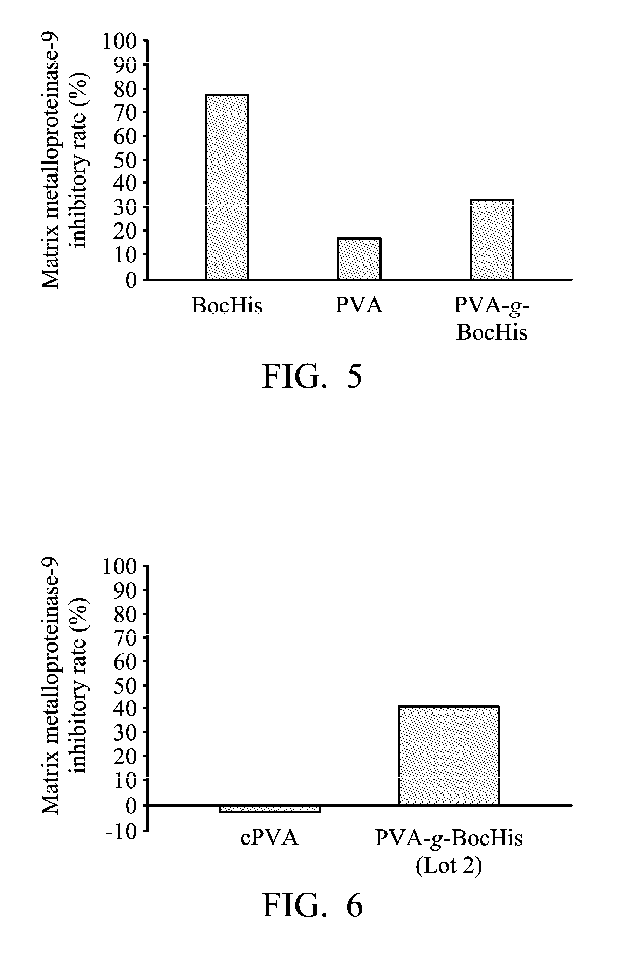

Powder of Boc-histidine grafted-polyvinyl alcohol derivative, "PVA-g-BocHis" obtained from Lot 1 of Example 1 (grafting ratio: 13.3%) was added to DMAC, stirred and dissolved at room temperature (500 rpm, 6 hours), and placed in a mold after being dissolved and then dried at 60°C for 72 hours to form a film. The film was cut into a film sample with a diameter of 1 cm and a 24 hour matrix metalloproteinase-9 activity test was performed thereto, and BocHis and PVA film were used as control groups. The results are shown in Table 6 and FIG. 5.

**Table 6: Inhibitory effect of a film formed from Boc-histidine grafted-polyvinyl alcohol derivative on matrix metalloproteinase-9**

| Sample | BocHis | PVA film | PVA-*g*-BocHis film (grafting ratio: 13.3%) |
|---|---|---|---|
| Matrix metalloproteinase-9 inhibitory rate (%) | 76.81 | 16.89 | 33.27 |

The results showed that the film of Lot 1 could reach an inhibitory rate of 33.27% to matrix metalloproteinase-9 while PVA was dissolved slowly and only has 16.89% of activity decreasing to matrix metalloproteinase-9.

D. Evaluation of inhibitory effect of a film of histidine grafted-polyvinyl alcohol derivative, "PVA-g-BocHis", of Lot 2 of Example 1 on matrix metalloproteinase-9 activity.

Powder of histidine grafted-polyvinyl alcohol derivative, "PVA-g-BocHis" obtained from Lot 2 of Example 1 (grafting ratio: 19.0%) was added to DMAC, stirred and dissolved at room temperature (500 rpm, 6 hours), and placed in a mold after being dissolved to dry at 60°C for 72 hours to form a film. The film was cut into a film sample with a diameter of 1 cm and a 24 hour matrix metalloproteinase-9 activity test was performed thereto, and a crosslinked PVA film (cPVA) was used as control groups. The results are shown in Table 7 and FIG. 6.

**Table 7: Inhibitory effect of a film formed from Boc-histidine grafted-polyvinyl alcohol derivative on matrix metalloproteinase-9**

| | Matrix metalloproteinase-9 inhibitory rate (%) | Standard deviation (%) | Boc-Histidine (mg/ml) |
|---|---|---|---|
| cPVA film | -2.34 | 5.48 | - |
| PVA-g-BocHis film | 41.04 | 0.84 | 19.40 |

| | | | |
|---|---|---|---|
| -: containing no histidine | | | |

Since the area of the film was constant, according to the weight and grafting ratio of the film, it was known that the concentration of the grafted histidine derivative of Lot 2 of Example 1 corresponding to the matrix metalloproteinase-9 activity analysis buffer was about 19.4 mg/ml, and the film of Lot 2 could reach an inhibitory rate of 41.04% to matrix metalloproteinase-9. Comparatively, the crosslinked PVA film (cPVA) had no activity inhibiting effect on matrix metalloproteinase-9.

E. Evaluation of inhibitory effect of a film of Boc-histidine grafted-polyvinyl alcohol derivative, "PVA-g-BocHis", of Lot 1 of Example 1 on matrix metalloproteinase-9 activity

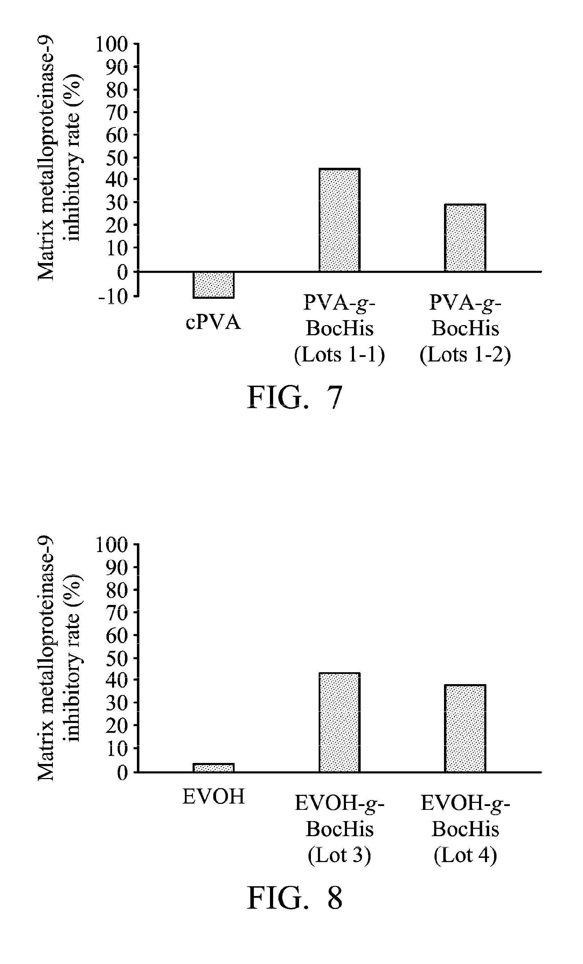

Powder of Boc-histidine grafted-polyvinyl alcohol derivative, "PVA-g-BocHis" obtained from Lot 1 of Example 1 (grafting ratio: 13.3%) was added to DMAC, stirred and dissolved at room temperature (500 rpm, 6 hours), and placed in a mold after being dissolved to dry at 60°C for 72 hours to form a film. The film was cut into a film sample with a diameter of 1 cm and a 24 hour matrix metalloproteinase-9 activity test was performed thereto, and a crosslinked PVA film (cPVA) was used as a control groups. The results are shown in Table 8 and FIG. 7.

**Table 8: Inhibitory effect of a film formed from Boc-histidine grafted-polyvinyl alcohol derivative on matrix metalloproteinase-9**

| | Matrix metalloproteinase-9 inhibitory rate (%) | Standard deviation (%) | Boc-Histidine (mg/ml) |
|---|---|---|---|
| cPVA film | -11.06 | 3.71 | - |
| PVA-g-BocHis film Lot 1-1 | 44.97 | 3.66 | 21.60 |
| PVA-g-BocHis film Lot 1-2 | 29.26 | 4.53 | 12.40 |

| | | | |
|---|---|---|---|
| -: containing zero Boc-histidine | | | |

Since the area of the film was constant and lot difference resulted in thickness difference between lots of PVA-g-BocHis samples, Lot 1-1 and Lot 1-2, according to the weight and grafting ratio of the film, it was known that the concentration of the grafted histidine derivative of PVA-g-BocHis films Lot 1-1 and Lot 1-2 corresponding to the matrix metalloproteinase-9 activity analysis buffer were about 21.64 mg/ml and 12.4 mg/ml, respectively. According to the results, it was known that Lot 1-1 and Lot 1-2 films had inhibitory rates of 29.26% and 44.97% on matrix metalloproteinase-9 activity, respectively. Comparatively, the crosslinked PVA film (cPVA) had no activity inhibiting effect on matrix metalloproteinase-9.

F. Evaluation of inhibitory effect of a film of Boc-histidine grafted-poly(ethylene vinyl-co-alcohol) derivative, "EVOH-g-BocHis", of Lot 3 and Lot 4 of Example 2 on matrix metalloproteinase-9 activity.

Powder of Boc-histidine grafted-poly(ethylene vinyl-co-alcohol) derivative, "EVOH -g-BocHis" obtained from Lot 3 and Lot 4 of Example 2 were added to DMAC, respectively, stirred and dissolved at room temperature (500 rpm, 6 hours), and placed in a mold after being dissolved to dry at 60°C for 72 hours to form films. The films were cut into film samples with a diameter of 1 cm and a 24 hour matrix metalloproteinase-9 activity test was performed thereto, and an EVOH film was used as a control groups. The results are shown in Table 9 and FIG. 8.

**Table 9: Inhibitory effect of a film formed from Boc-histidine grafted-poly(ethylene vinyl-co-alcohol) derivative on matrix metalloproteinase-9**

| | Matrix metalloproteinase-9 inhibitory rate (%) | Standard deviation (%) | Histidine (mg/ml) |
|---|---|---|---|
| EVOH film | 3.84 | 0.68 | - |
| EVOH-*g*-BocHis (Lot 3) film | 43.68 | 0.23 | 17.70 |
| EVOH-*g*-BocHis (Lot 4) film | 38.25 | 0.95 | 24.03 |

| | | | |
|---|---|---|---|
| -: containing no histidine | | | |

Since the area of the film was constant and lot difference resulted in a thickness difference between lots of EVOH-g-BocHis sample Lot 3 and Lot 4, according to the weight and grafting ratio of the film, it was known that the concentration of the grafted histidine of EVOH-g-BocHis films Lot 3 and Lot 4 corresponding to the matrix metalloproteinase-9 activity analysis buffer were about 17.7 mg/ml and 24.03 mg/ml, respectively. According to the results, it was known that activity inhibiting effect of Lot 3 and Lot 4 films had activity inhibiting rates of 43.68% and 38.25%, respectively, due to the increased histidine weight percentage. Lot 3 and Lot 4 films had inhibitory rates of 43.68% and 38.25% on matrix metalloproteinase-9 activity, respectively. Comparatively, the EVOH film had no activity inhibiting effect.

G. Evaluation of inhibitory effect of a film of Boc-histidine grafted-hydroxypropyl methylcellulose derivative, "HPMC-g-BocHis", of Lot 5 of Example 3 on matrix metalloproteinase-9 activity.

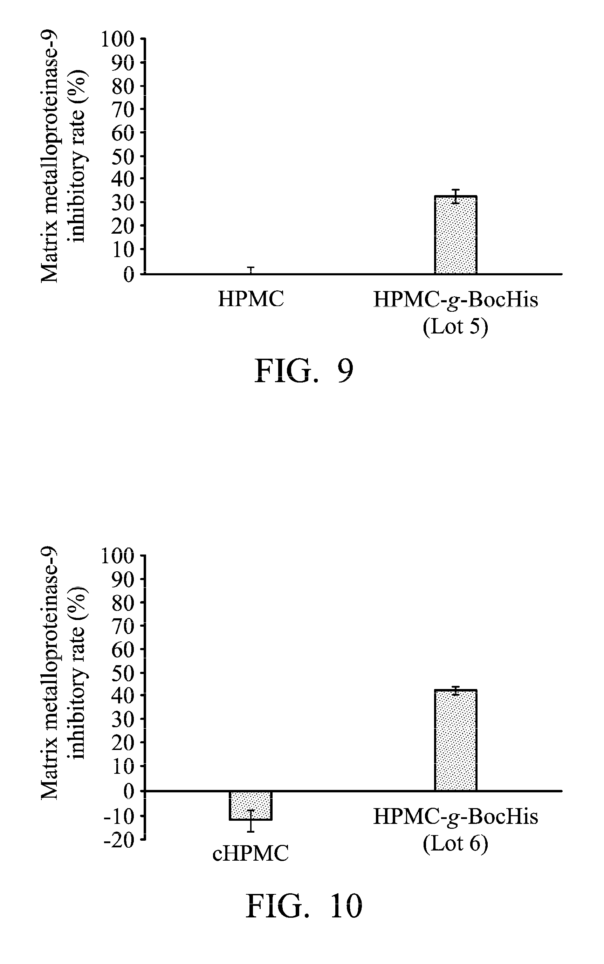

Powder of histidine derivative grafted-hydroxypropyl methylcellulose derivative, "HPMC -g-BocHis" obtained from Lot 5 of Example 3 was dissolved in DMAC, and then placed in a mold after being dissolved to dry at 60°C for 72 hours to form a film. The film was cut into a film sample with a diameter of 1 cm and a 3 hour matrix metalloproteinase-9 activity test was performed thereto, and an HPMC film was used as a control group. The results are shown in Table 10 and FIG. 9.

**Table 10: Inhibitory effect of a film formed from histidine derivative grafted- hydroxypropyl methylcellulose derivative on matrix metalloproteinase-9**

| | Matrix metalloproteinase-9 inhibitory rate (%) | Standard deviation (%) | Boc-Histidine (mg/ml) |
|---|---|---|---|
| HPMC film | 0 | 3.33 | - |
| HPMC-*g*-BocHis (Lot 5) film | 33.2 | 3.01 | 2.4 |

| | | | |
|---|---|---|---|
| -: containing no histidine | | | |

According to the weight and grafting ratio, it was known that the concentration of the grafted Boc-histidine of HPMC-g-BocHis Lot 5 film corresponding to the matrix metalloproteinase-9 activity analysis buffer was about 2.4 mg/ml. According to the results, it was known that Lot 5 film had an inhibitory rate of 33.2% on matrix metalloproteinase-9 activity. Comparatively, the HPMC film had no activity inhibiting effect.

H. Evaluation of inhibitory effect of a film of Boc-histidine grafted-hydroxypropyl methylcellulose derivative, "HPMC-g-BocHis", of Lot 6 of Example 3 on matrix metalloproteinase-9 activity.

Powder of Boc-histidine grafted-hydroxypropyl methylcellulose derivative, "HPMC -g-BocHis" obtained from Lot 5 of Example 3 was dissolved in DMAC, and then placed in a mold after being dissolved to dry at 60°C for 72 hours to form a film. The film was cut into a film sample with a diameter of 1 cm and a 24 hour matrix metalloproteinase-9 activity test was performed thereto, and a crosslinked HPMC (cHPMC) film was used as a control group. The results are shown in Table 11 and FIG. 10.

**Table 11: Inhibitory effect of a film formed from histidine derivative grafted- hydroxypropyl methylcellulose derivative on matrix metalloproteinase-9**

| | Matrix metalloproteinase-9 inhibitory rate (%) | Standard deviation (%) | Boc-Histidine (mg/ml) |
|---|---|---|---|
| CHPMC film | -12.69 | 4.06 | - |
| HPMC-g-BocHis (Lot 6) film | 42.41 | 1.89 | 10.15 |

| | | | |
|---|---|---|---|
| -: containing no Boc-histidine | | | |

According to the weight and grafting ratio, it was known that the concentration of the grafted histidine derivative of HPMC-g-BocHis Lot 6 film corresponding to the matrix metalloproteinase-9 activity analysis buffer was about 10.15 mg/ml. According to the results, it was known that Lot 6 film had an inhibitory rate of 42.41% on matrix metalloproteinase-9 activity. Comparatively, the CHPMC film had no activity inhibiting effect.

I. Evaluation of inhibitory effect of a film of Boc-histidine grafted-hydroxypropyl methylcellulose derivative, "HPMC-g-BocHis", of Lot 6 and Lot 7 of Example 3 on matrix metalloproteinase-9 activity.

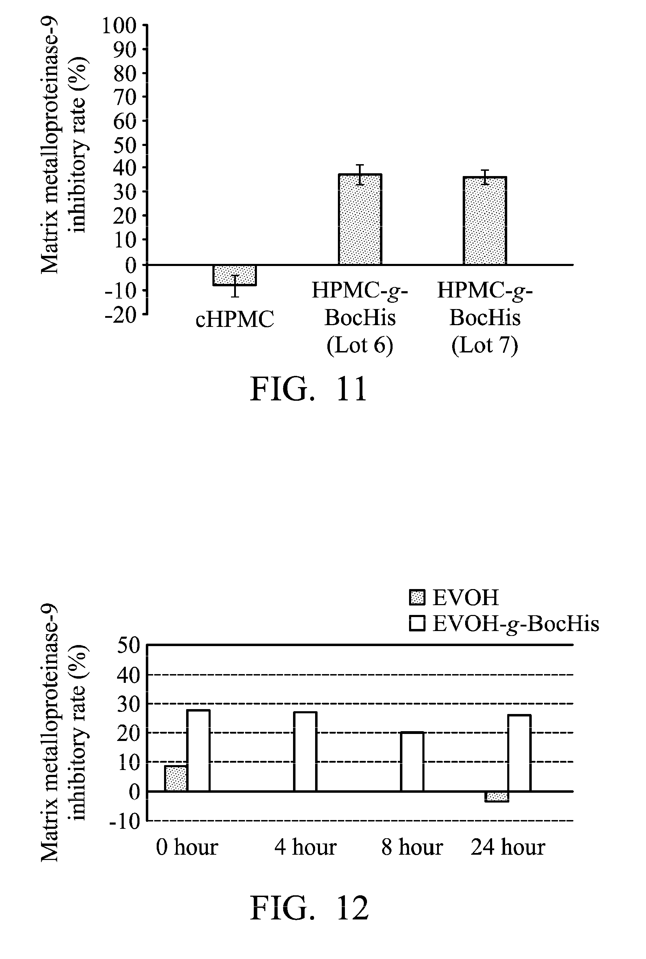

Powder of Boc-histidine grafted-hydroxypropyl methylcellulose derivative, "HPMC -g-BocHis" obtained from Lot 6 and Lot 7 of Example 3 were dissolved in DMAC, respectively, and then placed in a mold after being dissolved to dry at 60°C for 72 hours to form films. The films were cut into film samples with a diameter of 1 cm and a 2 hour matrix metalloproteinase-9 activity test was performed thereto, and a crosslinked HPMC (cHPMC) film was used as a control groups. The results are shown in Table 12 and FIG. 11.

**Table 12: Inhibitory effect of a film formed from histidine derivative grafted- hydroxypropyl methylcellulose derivative on matrix metalloproteinase-9**

| | Matrix metalloproteinase-9 inhibitory rate (%) | Standard deviation (%) | Boc-Histidine (mg/ml) |
|---|---|---|---|
| CHPMC film | -8.94 | 4.21 | - |
| HPMC-g-BocHis (Lot 6) film | 37.26 | 4.16 | 5.52 |
| HPMC-g-BocHis (Lot 7) film | 36.37 | 3.06 | 8.71 |

| | | | |
|---|---|---|---|
| -: containing no Boc-histidine | | | |

Since the area of the film was constant but lot difference resulted in thickness difference between Lot 6 and Lot 7, according to the weight and grafting ratio of the film, it was known that the concentration of the grafted histidine of HPMC-g-BocHis films Lot 6 and Lot 7 corresponding to the matrix metalloproteinase-9 activity analysis buffer were about 5.52 mg/ml and 8.71 mg/ml, respectively. According to the results, it was known that Lot 6 and Lot 7 films had activity inhibiting rates of 37.26% and 36.37% to matrix metalloproteinase-9, respectively since they were affected by the change of hydrophilic property. Comparatively, the crosslinked HPMC film (cHPMC) had no activity inhibiting effect.

### Example 7

### Experiment for evaluating long-term inhibiting effect

### Preparation for experimental samples

### A. Preparation for Boc-histidine grafted-poly(ethylene vinyl-co-alcohol) derivative ("EVOH -g-BocHis):

Powder of histidine derivative grafted-poly(ethylene vinyl-co-alcohol) of Lot 3 and Lot 4 were added to DMAC, respectively, stirred and dissolved at room temperature (500 rpm, 6 hours), and placed in a mold after being dissolved to dry at 60°C for 72 hours to form films.

B. Preparation for soaked film sample (BocHis coated EVOH Film, EVOH-c-BocHis).

Appropriate amount of Boc-Histidine was shaken in a 10 ml co-solvent (DMAc/MeOH 5:95) to be dissolved. After Boc-Histidine was completely dissolved, a poly(ethylene vinyl-co-alcohol) film was socked in the Boc-Histidine solution, and then taken out. After that, the film was placed in a 60°C oven for 24 hours to remove the solvent in the solution, and then a BocHis coated EVOH film is completed. Content of Boc-Histidine coated on EVOH film is determined as 9.18% by nuclear magnetic resonance spectroscopy and elemental determination analysis.

### Experimental method and results

A. Experiment for evaluating long-term inhibiting effect of Boc-histidine grafted-poly(ethylene vinyl-co-alcohol) derivative film sample (Lot 3) on matrix metalloproteinase-9.

The Boc-histidine grafted-poly(ethylene vinyl-co-alcohol) derivative film sample (Lot 3) was placed in PBS buffer at 37°C and shaken at 150 rpm, and the PBS buffer was replaced with fresh PBS buffer every 2 hours to mimic the physiological conditions for body fluid. At 4, 8 and 24 hours, a matrix metalloproteinase-9 activity test was performed to the Boc-histidine grafted-poly(ethylene vinyl-co-alcohol) derivative film sample. A poly(ethylene vinyl-co-alcohol) film was used as a control group. The results are shown in Table 13 and FIG. 12.

**Table 13: Results of an evaluation of long-term inhibiting effect of Boc-histidine grafted-poly(ethylene vinyl-co-alcohol) derivative film sample (Lot 3) on matrix metalloproteinase-9**

| | Matrix metalloproteinase-9 inhibitory rate (%) | | | |
|---|---|---|---|---|
| | 0 hour | 4 hour | 8 hour | 24 hour |
| EVOH film | 8.6 | 0 | 0 | -3.16 |
| EVOH-g-BocHis film | 27.84 | 27.02 | 20.39 | 26.09 |

According to Table 13 and FIG. 12, it is known that the inhibitory effect of the Boc-histidine grafted-poly(ethylene vinyl-co-alcohol) derivative film sample on matrix metalloproteinase-9 is evenly kept between 20.39% and 27.84%, effectively.

B. Evaluation for long-term inhibiting effect of only Boc-histidine socking treated substrate (EVOH-c-BocHis) and Boc-histidine grafted-poly(ethylene vinyl-co-alcohol) derivative film sample (Lot 4).

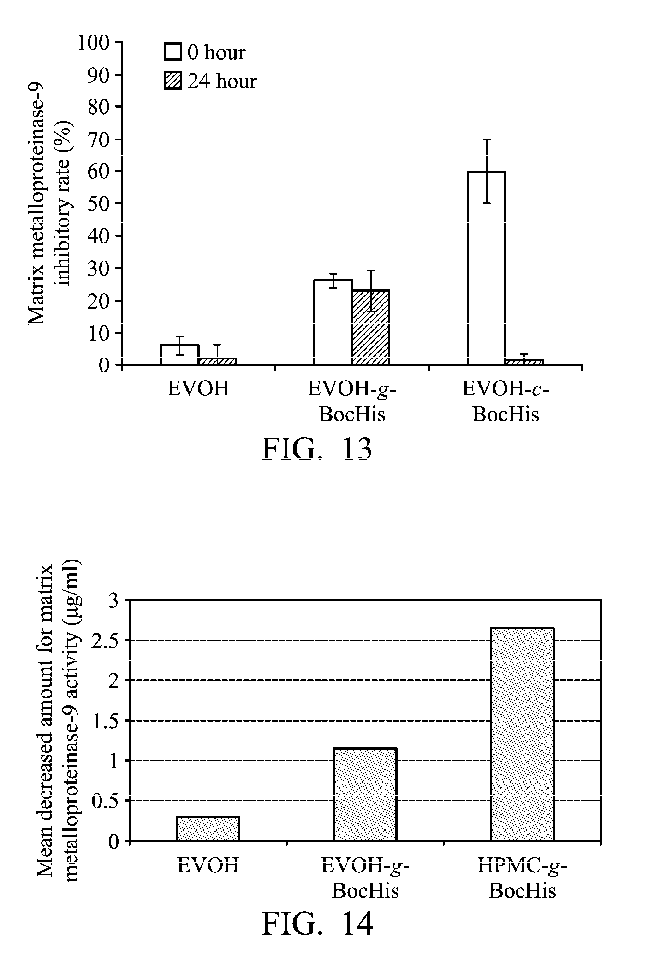

The Boc-histidine socking treated substrate (EVOH-*c*-BocHis) and Boc-histidine grafted-poly(ethylene vinyl-co-alcohol) derivative film sample (EVOH-*g*-BocHis, Lot 4) were placed in PBS buffer at 37°C and shaken at 150 rpm, respectively, and the PBS buffer was replaced with fresh PBS buffer every 2 hours to mimic the physiological conditions for body fluid. At the beginning and 24 hour, matrix metalloproteinase-9 activity test was performed to the histidine derivative socking treated substrate (EVOH-*c*-BocHis) and Boc-histidine grafted-poly(ethylene vinyl-co-alcohol) derivative film sample (EVOH-*g*-BocHis, Lot 4), respectively. The results are shown in Table 14 and FIG. 13.

**Table 14: Results of an evaluation of long-term inhibiting effect of only histidine socking treated substrate (EVOH-c-BocHis) and histidine grafted-poly(ethylene vinyl-co-alcohol) derivative film sample (Lot 4)**

| Sample | EVOH | | EVOH-*g*- BocHis | | EVOH-*c*-BocHis | |
|---|---|---|---|---|---|---|
| Time | 0 hour | 24 hour | 0 hour | 24 hour | 0 hour | 24 hour |
| Matrix metalloproteinase-9 inhibitory rate (%) | 6.01±2.84 | 7.01±5.7 | 26.18±2.19 | 22.87±6.4 | 66.91±10.14 | 1.51±1.8 |

The result showed that the Boc-histidine grafted-poly(ethylene vinyl-co-alcohol) derivative film sample (Lot 4) not only had inhibitory effect on matrix metalloproteinase-9 but also could further retain the inhibitory effect for a long term. On the contrary, EVOH-c-BocHis which coated with 9.18 wt% BocHis had a good inhibiting effect on matrix metalloproteinase-9 at first (66.91%), however it lost nearly all the inhibiting effect after 24 hours.

### Example 8

Examination of activity of matrix metalloproteinase in wound fluid of diabetic rat.

### Preparation for experimental samples

A. Preparation for Boc-histidine grafted-poly(ethylene vinyl-co-alcohol) derivative (EVOH-g-BocHis) film sample:

Powder of Boc-histidine grafted-poly(ethylene vinyl-co-alcohol) derivative of Example 2 were added to DMAC with a proportion of 15% solid content, stirred and dissolved at room temperature, and placed in a mold after being dissolved to dry at 60°C for 72 hours to form a film.

B. Preparation for Boc-histidine grafted-hydroxypropyl methylcellulose derivative (HPMC-g-BocHis) film sample:

Powder of Boc-histidine grafted-hydroxypropyl methylcellulose derivative of Example 3 were added to DMAC with a proportion of 15% solid content, stirred and dissolved at room temperature, and placed in a mold after being dissolved to dry at 60°C for 72 hours to form a film.

C. Diabetic rat; Rats (Sprague-Dawley (SD)) were continuously injected with streptozotocin (STZ) for 4 weeks to result in animal models in a condition similar to type 2 diabetes.

### Experimental method and results

A. The backs of diabetic rats numbered 1 to 6 were each cut to form a wound with a size of 5 cm x 6 cm. The rats numbered 1 and 2 belonged to the EVOH treatment group, the rats numbered 3 and 4 belonged to the Boc-histidine grafted-poly(ethylene vinyl-co-alcohol) derivative (EVOH-g-BocHis) treatment group, and the rats numbered as 5 and 6 belonged to the Boc-histidine grafted-hydroxypropyl methylcellulose derivative (HPMC-*g-*BocHis).

B. The sample film was covered on the surface of the wound, and a waterproof breathable dressing was further covered on the sample film, and the dressing as fixed around the wound by an operating suture line. On day 1 and day 3 after the operation, the wound fluid was drawn out, and activity test was performed to matrix metalloproteinase-9 in the wound fluid.

### C. Matrix metalloproteinase-9 activity analysis:

The wound fluid was diluted with matrix metalloproteinase-9 activity analysis buffer (50mM Tris, 10 mM CaCl₂, 150 mM NaCl, 0.05% Brij-35(w/v), pH 7.5) for 50 folds. The wound fluid was taken and added to an equal volume of substrate (final concentration 10 uM) for matrix metalloproteinase-9, placed in a 37°Cincubator for reaction for 0.5-1 hour, and then the fluorescence (Ex/Em=320/405 nm) of the reaction was determined by a high sensitivity fluorescence reader (Flexstation3).

**Table 15: Results of an evaluation of the effect of Boc-histidine grafted derivative film samples on activity of matrix metalloproteinase-9 in the wound fluid**

| | Matrix metalloproteinase-9 activity (ug/mL) | | | | | |
|---|---|---|---|---|---|---|
| Sample | EVOH | | EVOH-*g*-BocHis | | HPMC-*g*-BocHis | |
| Rat number | 1 | 2 | 3 | 4 | 5 | 6 |
| Day 1 | 5.84 | 5.53 | 7.15 | 5.21 | 3.04 | 3.43 |
| Day 3 | 4.04 | 6.72 | 6.33 | 3.70 | 1.17 | -0.01 |
| Decreased amount | 1.8 | -1.19 | 0.82 | 1.51 | 1.87 | 3.44 |
| Mean decreased amount | 0.305 | | 1.165 | | 2.655 | |

According to Table 15 and FIG. 14, it was known that on day 3 after the operation, for the Boc-histidine grafted-poly(ethylene vinyl-co-alcohol) derivative (EVOH-g-BocHis) treatment group and the Boc-histidine grafted- hydroxypropyl methylcellulose derivative (HPMC-*g*-BocHis) treatment group, the activities of matrix metalloproteinase-9 in the wound fluid thereof were significantly lower than those on day 1, wherein the histidine grafted-hydroxypropyl methylcellulose derivative (HPMC-g-BocHis) had a better effect.

It will be apparent to those skilled in the art that various modifications and variations can be made to the disclosed embodiments. It is intended that the specification and examples be considered as exemplary only, with a true scope of the disclosure being indicated by the following claims and their equivalents.

## Claims

1. A polymer composition, comprising
a polymer having a hydroxyl group; and
a histidine or a histidine derivative grafted to the polymer having a hydroxyl group.

2. The polymer composition as claimed in claim 1, wherein the histidine or the histidine derivative accounts for about 0.1 to 99 wt% of the polymer composition.

3. The polymer composition as claimed in claim 1 or 2, wherein the polymer having a hydroxyl group comprises a synthetic polymer or a natural polymer.

4. The polymer composition as claimed in claim 3, wherein the synthetic polymer or the natural polymer comprises a linear polymer or a branched polymer.

5. The polymer composition as claimed in claim 3, wherein the synthetic polymer is a linear synthetic polymer.

6. The polymer composition as claimed in claim 5, wherein the linear synthetic polymer comprises polyalkylene glycol, polyvinyl alcohol (PVA), polyvinyl acetate (PVAc), poly(vinyl alcohol-co-vinyl acetate), poly(ethylene vinyl-co-alcohol), (EVOH) or a combination thereof.

7. The polymer composition as claimed in claim 3, wherein the natural polymer comprises a polysaccharide polymer.

8. The polymer composition as claimed in claim 7, wherein the polysaccharide polymer comprises hyaluronic acid, starch, cellulose, methylcellulose, hydroxypropyl cellulose, hydroxypropyl methylcellulose, oxidized cellulose, dextran, scleroglucan, chitin, chitosan, curdlan, alginate, carrageenan, pectin, gum Arabic, guar gum, gellan, pullulan, chondroitin sulfate, heparin or keratin sulfate.

9. The polymer composition as claimed in any one of claims 1-8, wherein the histidine derivative is a *N*^{α}-protected histidine derivative.

10. The polymer composition as claimed in claim 9, wherein the *N*^{α}-protected histidine derivative comprises *N*^{α}-Boc-histidine, *N*^{α}-Cbz-histidine, *N*^{α}-Fmoc-histidine or *N*^{α}-Ac-histidine.

11. The polymer composition as claimed in any one of claims 1-10, wherein the histidine or the histidine derivative is directly grafted to the polymer having a hydroxyl group, or is grafted to the polymer having a hydroxyl group by a spacer.

12. The polymer composition as claimed in claim 11, wherein the histidine or the histidine derivative is directly grafted to the polymer having a hydroxyl group.

13. The polymer composition as claimed in claim 12, wherein the histidine or the histidine derivative is directly grafted to the polymer having a hydroxyl group through a chemical covalent bond.

14. The polymer composition as claimed in claim 13, wherein the chemical covalent bond comprises an ester bond or a urethane.

15. The polymer composition as claimed in claim 12, wherein the polymer having a hydroxyl group is polyvinyl alcohol (PVA), poly(ethylene vinyl-co-alcohol) (EVOH), hyaluronic acid, cellulose or hydroxypropyl methylcellulose, and the histidine derivative is *N*^{α}-Boc-histidine.

16. The polymer composition as claimed in claim 11, wherein the histidine or the histidine derivative is grafted to the polymer having a hydroxyl group by the spacer.

17. The polymer composition as claimed in claim 16, wherein the spacer comprises alkyl carbon chains, phenyl group, polyethyleneoxide (PEO), polyethylglycol (PEG), or polypropylene oxide (PPO) repeating units.
